# EUROPEAN PATENT SPECIFICATION

(11) **EP 2 511 272 B1**
(45) Date of publication and mention of the grant of the patent: **15.04.2015**
(21) Application number: 12001973.2
(22) Date of filing: 21.03.2012
(51) Int. Cl.: C07D 303/04, A61K 31/336, A61P 29/00

(54) **Synthetic analogues of abscisic acid with anti-inflammatory and insulin release stimulation effects on human cells**
Synthetische Analoga von Abscisinsäure mit einer entzündungshemmenden Wirkung und Insulin Stimulierung in menschlichen Zellen
Analogues synthétiques de l'acide abscissique avec un effet anti-inflammatoire et de stimulation de l'insuline dans les cellules humaines.

(30) Priority: 22.03.2011 IT MI20110450
(43) Date of publication of application: 17.10.2012
(73) Proprietor: Universita' degli Studi di Genova, 16126 Genova (IT)
(72) Inventor: Zocchi, Elena, 16166 Genova (IT); De Flora, Antonio, 16146 Genova (IT); Millo, Enrico, 16131 Genova (IT); Bruzzone, Santina, 16011 Arenzano (Genova) (IT); Guida, Lucrezia, 16136 Genova (IT); Grozio, Alessia, 16143 Genova (IT); Salis, Annalisa, 17047 Quiliano (SV) (IT); Magnone, Mirko, 16142 Genova (IT)
(74) Representative: Jaumann, Paolo

(56) References cited:
- JINMING ZHOU ET AL: "Synthesis and in vitro characterization of ionone-based chalcones as novel antiandrogens effective against multiple clinically relevant androgen receptor mutants", INVESTIGATIONAL NEW DRUGS ; THE JOURNAL OF NEW ANTICANCER AGENTS, KLUWER ACADEMIC PUBLISHERS, BO, vol. 28, no. 3, 24 April 2009 (2009-04-24) , pages 291-298, XP019788472, ISSN: 1573-0646
- UENO K ET AL: "Differences between the structural requirements for ABA 8'-hydroxylase inhibition and for ABA activity", BIOORGANIC & MEDICINAL CHEMISTRY, PERGAMON, GB, vol. 13, no. 10, 16 May 2005 (2005-05-16), pages 3359-3370, XP004859472, ISSN: 0968-0896, DOI: DOI:10.1016/J.BMC.2005.03.015
- ARAI S ET AL: "Synthesis and biological activity of 3'-chloro, -bromo, and iodoabscisic acids, and biological activity of 3'-fluoro-8'-hydroxyabscisic acid", PHYTOCHEMISTRY, PERGAMON PRESS, GB, vol. 52, no. 7, 1 December 1999 (1999-12-01), pages 1185-1193, XP004291210, ISSN: 0031-9422, DOI: DOI:10.1016/S0031-9422(99)00444-6
- PERRAS M ET AL: "Defining steric, electronic and conformational requirements of carrier-mediated uptake of abscisic acid in barley suspension culture cells", PHYTOCHEMISTRY, PERGAMON PRESS, GB, vol. 46, no. 2, 1 September 1997 (1997-09-01), pages 215-222, XP004293425, ISSN: 0031-9422, DOI: DOI:10.1016/S0031-9422(97)00299-9
- BALSEVICH J J ET AL: "Activity and utility of abscisic acid having a 3' thioether linker arm", PHYTOCHEMISTRY, PERGAMON PRESS, GB, vol. 44, no. 2, 1 January 1997 (1997-01-01), pages 215-220, XP004292773, ISSN: 0031-9422, DOI: DOI:10.1016/S0031-9422(96)00440-2
- ALEU ET AL.: "Lipase-mediated synthesis of the enantiomeric forms of 4,5-epoxy-4,5-dihydro-alpha-ionone and 5,6-epoxy-5,6-dihydro-beta-ionone. A new direct access to enantiopure (R)- and (S)-alpha-ionone", J. CHEM. SOC. PERKIN TRANS. 1, 1 January 1999 (1999-01-01), pages 271-278,

## Description

### DISCLOSURE

The present invention relates to a novel class of compounds having anti-inflammatory activity. In particular, to a novel class of compounds having abscisic acid antagonist activity in human cells. According to a particular aspect of the invention, the compounds can be used for treatment and prevention of inflammatory, in particular chronic inflammatory, states, atherosclerosis and metabolic syndrome.

Abscisic acid (± 2-cis, 4-trans abscisic acid, simply referred to herein in the following as "ABA"), known as a vegetable hormone of basic importance in plant physiology, was recently found to be an endogenous hormone in animals, with pro-inflammatory activity. In particular, it was discovered that ABA is produced and released by human and murine inflammatory cells (granulocytes, monocytes, microglia) and that it stimulates pro-inflammatory functions of these cells (migration, release of reactive species of oxygen and of cytokines, phagocytosis). ABA produced by activated monocytes / macrophages stimulates, by paracrine stimulation, the proliferation of vascular smooth muscle cells. ABA is furthermore present in human atherosclerotic plaques at 10-times higher concentrations than in healthy arterial tissue.

ABA is also produced by adipose tissue and human and murine β-pancreatic cells stimulated by glucose: ABA enhances glucose-dependent, and stimulates glucose-independent, insulin release. Furthermore, ABA concentration in plasma (1 nM) increases in healthy individuals after glucose administration.

ABA shows hence the capability of both influencing the activation of inflammatory cells and energetic metabolism. On one hand, ABA appears to play a central role in exchange of paracrine signals between inflammatory cells and VSMC, which activates functional responses implied in atherosclerosis. On the other hand, it would appear that ABA produced by inflammatory cells and adipose tissue has an influence on release of insulin and consequently plays a role in the pathogenesis of atherosclerosis and type 2 diabetes (T2D), in particular in the presence of obesity.

In the past, the human receptor of ABA (LANCL-2) was identified and a cellular line overexpressing ABA's signal transduction system (receptor and second messengers) was developed, for use for screening of naturally occurring and synthetic compounds in the search for ABA antagonists. This cellular line was the subject-matter of patent application WO 2010/015646.

Further reference is made to Ueno K. et al., "Bioorganic & Medicinal Chemistry", vol. 13(10), 2005, pages 3359-3370.

A new class of compounds active as ABA antagonists in human cells in particular as regards the anti-inflammatory effect of ABA, has now been discovered. Surprisingly, the compounds which are the subject-matter of the present invention result also to stimulate insulin release by human and murine cells. Said compounds have the following formula: 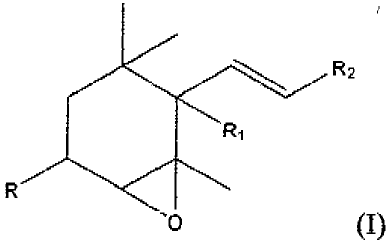 wherein R is selected from hydrogen and =O groups, R1 is selected from -H and -OH, R2 is selected from groups having one of following structural formulae 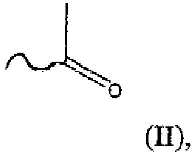 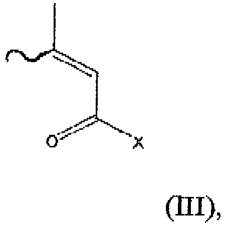 wherein X is selected from -OH, alkylamino-, arylamino-, alkylarylamino- or arylalkylamino-, alkoxy-, aryloxy-, alkylaryloxy- or arylalkyloxy- groups, with said groups preferably having one to eight carbon atoms and being optionally substituted; the invention relates also to pharmaceutically acceptable salts of said compounds. Such groups may contain atoms or functionalities different from C, in particular keto, carboxy, ether, amino functionalities. The alkyl chains of said groups may optionally be branched or may form rings, or may contain unsaturations.

A particularly preferred compound is the compound corresponding to formula (I), in which R is hydrogen, R1 is H, R2 corresponds to formula (II), i.e., 4,5-epoxy-4,5-dihydro-alpha-ionone. The nomenclature and numbering of said compounds, as used herein, are used in the literature, for example in "Journal of the Chemical Society, Perkin Transactions 1: Organic and Bio-Organic Chemistry, (3), 271-278; 1999". Alternative names which can be found in literature are 3-buten-2-one, 4-[(1S,2S,6R)-1,3,3-trimethyl-7-oxabicyclo[4.1.0]hept-2-yl]-, (3E)- and 3-buten-2-one, 4-[(1R,2R,6S)-1,3,3-trimethyl-7-oxabicyclo[4.1.0]hept-2-yl]-, (3E)-; mixtures of both isomers have been used according to a possible aspect of the present invention. Another particularly preferred compound corresponds to formula (1), in which R is =O, R1 is -OH, R2 corresponds to formula (III) wherein X is -OH, i.e., 2'α,3'α-dihydro-2'α,3'α-epoxyabscisic acid.

Other preferred compounds correspond to formula (I), in which R is =O, R1 is -OH, R2 corresponds to formula (III) wherein X is selected from -OCH₃, -NHCH₃, -N(CH₂CH₃)₂,

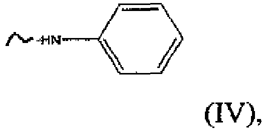

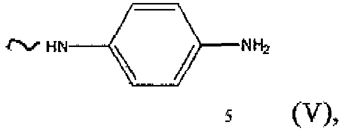

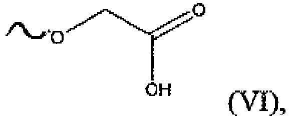

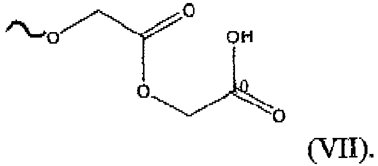

Relatively to the C-C double bond contained in formula (I), the substituents are preferably arranged in E configuration, relatively to the C-C double bond contained in formula (III), the substituents are preferably arranged in Z configuration, analogously to the configuration which is seen in naturally occurring ABA.

As regards the epoxy functionality in the compounds, two possible spatial configurations exist, which are designated as "cis" and "trans" configurations. According to a possible aspect, both of them can be used. According to a further aspect of the invention, the trans configuration is preferred, in particular in case of 2'α, 3'α-dihydro-2'α,3'α-epoxyabscisic acid, with the trans isomer being particularly preferred. The compounds can be used as variable mixtures of various isomers, in particular those mixtures in such ratios as deriving from the production process used. According to a further aspect, the isomers can be separated, in order to use those regarded as suitable in purity conditions.

As regards the expression "pharmaceutically acceptable salts", it refers to salts with no or acceptable toxicity of the compounds according to the present invention, obtained by addition of inorganic or organic acids or bases. In the latter case, which is interesting in particular when the compound, as e.g., in case of 2'α, 3'α -dihydro-2'α, 3'α -epoxyabscisic acid, as well as of other derivatives, contains acidic functionalities, examples are metal salts, as salts of alkali metals, alkali-earth metals, transition metals (for example, Na, K, Al, Ca, Mg), of ammonium or of primary, secondary and tertiary amines (for example. triethylamine, diethylamine, morpholine, piperidine, piperazine) or with basic amino acids, oxamines (e.g.. meglumine), amino alcohols (e.g. 3-aminobutanol or 2-aminoethanol). In the first case, if the compound contains basic functionalities, the salts can be of mineral or organic acids (e.g., hydrochlorides, hydrobromides, sulfates, hydrogen sulfates, dihydrogen sulfates, citrates, malates, fumarates, trifluoroacetates, 2-naphthalenesulfonates, p-toluenesulfonates).

Also hydrates or solvates of the above mentioned compounds fall within the claimed scope of protection.

The compounds according to the present invention can be easily synthesized from ABA available on the marked, or from α-ionone.

According to a possible aspect, racemic mixtures are used in case of both starting compounds. The obtained compounds can be mixtures of different stereoisomers, and be used as such, or, if regarded as suitable, said mixtures can be submitted to separation, e.g., by liquid chromatography.

Alternatively, a specific stereoisomer of the starting compound can be used, for example the (+)ABA isomer, which is the predominating isomer in natural environment. Said synthesis can be followed, or not, as regarded suitable, by a separation of the formed isomers.

The invention is illustrated in greater detail in the following with reference to preferred, not exclusive, embodiments.

The compounds of the present invention result to be ABA inhibitors in human granulocytes and monocytes. These molecules additionally behave as inhibitors of the increase in concentration of cyclic AMP (cAMP, the second messenger of ABA) induced by 1 µM ABA in human granulocytes.

Competition-binding experiments indicate the capability of displacing the binding of 10 nM radioactive ABA {[³H]-ABA} from the membrane of human granulocytes at concentrations of compound according to the present invention lower than ABA (EC₅₀ = 0.87 nM).

Furthermore, an antagonist activity is observed of ABA pro-inflammatory effects *in vitro* on human granulocytes and monocytes. In particular, in human granulocytes, an inhibition resulted of ABA-induced chemotaxis, of ABA-induced production of oxygen radicals, of ABA-induced phagocytosis and of ABA-induced production of prostaglandin E2 (PGE2). Furthermore, the ABA-induced production of pro-inflammatory molecules PGE2 and monocyte chemoattractant protein-1 (MCP-1) results to be inhibited in human monocytes.

Surprisingly, on considering their above evidenced ABA antagonist activity, the compounds according to the present invention showed the capability of stimulating the release of insulin by human beta pancreatic cells and by rat insulinoma lines. At nanomolar concentrations, the stimulation is observed of insulin release by INS-1 cells and by human beta pancreatic cells, both at low and high glucose concentrations, in a similar way to ABA from a quantitative point of view, at a same concentration. Furthermore, the effects of ABA and of the compounds according to the present invention are mutually potentiating each other, both at low and high glucose concentrations.

In cells silenced for the espression of LANCL-2, the receptor of ABA, (Sturla L, Fresia C, Guida L, Grozio A, Vigliarolo T, Mannino E, Millo E, Bagnasco L, Bruzzone S, De Flora A, Zocchi E. Binding of abscisic acid to human LANCL2. Biochem Biophys Res Commun. 2011 Nov 18th;415(2):390-5), ABA results to be inactive, both at low and high glucose concentrations. On the contrary, surprisingly, the compounds according to the present invention result to keep alive the stimulating activity of insulin secretion. This observation suggests the hypothesis that the effect shown by the compounds according to the present invention on insulin-secreting cells be mediated by a intracellular signalling mechanism different from ABA. This conclusion is also supported by the matter of fact that the effects of ABA and of a compound selected form among those of the present invention reciprocally enhance each other when both compounds are used together.

Without willing to be bound to any specific theories, the observation that the compounds are capable of antagonizing ABA binding to LANCL-2 suggests that this compound would activate an intracellular signalling mechanism different form ABA's in insulin-secreting cells: these cells could express, besides to LANCL-2, a further receptor for ABA-similar molecules, which, in particular, is activated by the compounds according to the present invention. Or, LANCL-2 could be coupled to two different signal transducing machines in insulin-secreting cells: a first one, common to inflammatory cells and another one mediated by other molecular components (G protein, protein kinase, second messengers). The conformational modification induced by the binding of compounds according to the present invention fo LANCLS-2 might therefore enable the activation of such a receptor, consequently enabling it to transduce the signal induced by such compounds binding.

Finally, an adequate stability was detected of the compounds of the present invention, both in aqueous solution and in plasma, as well as absence of cytotoxicity and cytostatic activity at concentrations of interest on human granulocytes and several human and murine cellular lines.

The invention relates also to a pharmaceutical compositions comprising a compound as defines above, and one o more pharmaceutically acceptable excipients.

The invention relates also to the use of a compound as defined above, for preparing medicaments, in particular for treatment of atherosclerosis, metabolic syndrome, chronic inflammatory pathologies and to the therapeutical use of said compound, in particular for the treatment of the above said pathologies. According to a further aspect, the invention relates also to the use of a compound as defined above, for preparing medicaments for treatment of diabetes, in particular type 2 diabetes.

The properties of the compounds according to the present invention are illustrated in greater detail in the following by means of the following examples, supplied for merely illustrative purposes, non limitative of the scope of protection of the accompanying claims.

### Example 1 - Synthesis of 2'α,3'α-dihydro-2'α,3'α-epodxyabscisic acid

Thirty (30) mg of *rac* (2Z,4E)-5-(1-hydroxy-2,6,6-trimethyl-4-oxo-2-cyclohexen-1-yl)-3-methyl-2,4-pentadienoic acid), viz. ABA (Sigma Aldrich) (0,113 mmol; 1 eq.) are dissolved in 2 ml of methanol and the resulting mixture is kept under magnetic stirring a T =0°C. Two hundred and fifty (250) µl of H₂O₂ (8.09 mmol; 70 eq) and 50 µl of 6N sodium hydroxide are added to the solution, which is then kept under stirring at T = 4°C and in the dark for 24 hours. The reaction is monitored by TLC (hexane:ethyl acetate:acetic acid 50:35:1) and HPLC. When the reaction is ended, the obtained solution is mixed with ammonium hydrogen carbonate and is extracted with ethyl acetate (3 x 3 ml). The organic phase is then concentrated *in vacuo,* with a thick oil being obtained, which is purified by HPLC.

The molecular structure was confirmed by mass spectrometry and NMR analysis.

### Example 2 - Synthesis of 4,5-epoxy-4,5-dihydro-α-ionone

A solution of 600 µl of dichloromethane and 11 µl di of (R,S)-3-(E)-buten-2-one,4-(2,6,6-trimethyl-2-cyclohexen-1-yl), or α-ionone (Sigma Aldrich) (0.052 mmol; 1 eq) is kept under magnetic stirring at T = 0° C. To said solution 13.4 mg of m-chloro-perbenzoic acid (0.078 mmol; 1.5 eg) are added, and stirring is continued in the dark and at room temperature for eight hours.

The reaction is monitored by TLC (hexane:ethyl acetate:acetic acid 50:35:1).

When the reaction is ended, the obtained solution is mixed with ammonium hydrogen carbonate and is extracted with ethyl acetate (3 x 3 ml). The organic phase is then concentrated to a small volume *in vacuo,* with a thick oil being obtained, which is purified by HPLC.

The molecular structure was confirmed by mass spectrometry and NMR analysis.

### Example 3 - Determination of intracellular cAMP levels with compounds prepared according to examples 1 and 2.

Human granulocytes, after being incubated overnight (ON) in DMEM/F12 supplemented with 10% of foetal bovine serum (FCS), penicillin (50 units/ml) and streptomycin (50 µg/ml) (DMEM/F12 complete), were centrifuged at 200xg for 5 min and were then resuspended in HBSS (Hanks' Balanced Salt Solution) with calcium and magnesium (15 x 10⁶ cells /200 µl) and preincubated for 5 minutes at 25 °C in the presence of 10 µM of phosphodiesterase inhibitor 4-[(3-butoxy-4-methoxyphenyl)-methyl]-2-imidazolidinone (Ro-20-1724). Two hundred (200) µl of suspension were then drawn and were incubated for 1 minute with 1 µM ABA (Sigma-Aldrich) either alone, or in the presence of decreasing concentrations (from 50 µM to 0.01 nM) of test compounds obtained according to the preceding examples. The reaction was terminated by the addition of 13 µl of perchloroacetic acid (PCA) to the end concentration of 0.6 M at 4 °C.

The cell lysates were centrifuged for 5 min at 17,000 x g at 4 °C and the supernatant was adjusted at pH 7.5 with 3 M potassium carbonate (K₂CO₃). The reaction led to the development of gas CO₂ and the formation of a white precipitate of potassium perchlorate (KClO₄), which was removed by a 5-minute centrifugation at 17,000xg at 4 °C.

Intracellular concentration of cAMP was determined by means of the competitive radioimmunological test [³H]cAMP assay system (Amersham, GE Healthcare), following manufacturer's instructions..

The experiments were carried out three times, in triplicate.

Tests were carried out on human granulocytes according to the above explained procedure, in which the production of cAMP was compared to a group of control cells in case of treatment with ABA 1 µM and with ABA 1 µM + tested compound 50 µM. In case of compound prepared in example 1, inhibition was of 100 %, in case of compound prepared in example 2, inhibition was of 96 %.

Finally, the compound of example 1 was submitted to separation by chromatography of cis-trans isomers of epoxy functionality.

In the above disclosed test, inhibittion was of 100 % for trans isomer.

Furthermore, the trans isomer showed effectiveness also at concentration of down to 0.1 nM, as summarized in the following table:

**Table 1**

| Concentration of compound from Example 1, trans | Inhibition % |
|---|---|
| 5 µM | 100% |
| 1 µM | 100% |
| 100 nM | 100% |
| 10 µM | 100% |
| 1 nM | 57% |
| 0.1 nM | 25% |
| 0.01 nM | 0% |

The tested compound inhibits the increase in cAMP concentration, induced by 1 µM ABA, with an IC₅₀ of about 1 nM in human granulocytes.

### Example 4 - Experiments of competition binding with the compound prepared according to example 1.

Human granulocytes (7 x 10⁶ per point), after ON incubation in complete DMEM/F12, were incubated in triplicate at 25 °C for 60 min in 100 µl of HBSS spiked with glucose 10 mM in the presence either of an excess of unlabeled ABA (1 mM) (in order to determine the non-specific binding rate) or of increasing concentrations of compound prepared according to example 1 (from 0,01 nM up to 100 µM). To each compound, 10 nM of [³H]-ABA (GE Healthcare) with specific activity of 98,000 cpm/pmole were added. At the end of incubation, the cells were centrifuged for 30 sec at 1700xg, the supernatant was removed and discarded, and the pellet was washed once wih an excess of HBSS in ice, and centrifuged for 30 sec at 17,000xg. The pellet was resuspended in 200 µl of de-ionized water and radioactivity of cells was determined after addition of scintillation cocktail in a β counter.

Specific binding was computed as the difference between the total binding of [³H]-ABA in the cells and the binding of [³H]-ABA in the presence of an excess of unlabeled ABA (1 mM) (non-specific binding).

The experiment was carried out five times in triplicate.

The data obtained is summarized in following table 2.

**Table 2**

| [] Compound of Ex. 1, free | Bound [³H]ABA (fmol/mg)±SD |
|---|---|
| 100 µM | 7±2 |
| 1 µM | 7±2 |
| 100 nM | 10±1 |
| 10 nM | 12±2 |
| 1 nM | 28±3 |
| 0.1 nM | 43±4 |
| 0.01 nM | 51±7 |

From the analysis of data, it resulted that the IC₅₀ of the compound (i.e., the concentrations of competitor ligand which displaces 50 % of radioligand specific binding) is of 0.87 nM (95 % confidence intervals: from 0.48 nM to 1.58 nM), whereas the inhibition constant Ki of the compound prepared according to example 1, obtained by applying Cheng-Prusoff equation: Ki= IC₅₀ /1+ ([ABA]/KDABA) with [ABA] =10 nM and K_{D} ABA = 11 nM (Yamazaki e al., 2003) resulted to be of 0.46 nM (95% confidence intervals: from 0.25 nM to 0.83 nM).

The low Kᵢ value obtained indicates a high affinity of ABA receptor for the compound.

### Example 5 - Inhibition of chemotaxis by compound prepared according to example 1.

After ON (overnight) incubation in complete DMEM/F12, granulocytes were washed in HBSS and then were resuspended at the concentration of 1x10⁷ cells/ml in chemotaxis buffer (HBSS:PBS:5% albumin; 39:16:1).

Chemotaxis was carried out on 96-well plates ChemoTx system microplates (Neuro Probe, Inc., Gaithersburg, MD, U.S.A.) with a polycarbonate filter with 3 µm-diameter pores. The compound prepared according to example 1 was diluted in chemotaxis buffer at various concentrations (5 µM, 100 nM, 1 nM, 0.1 nM) and was added, either alone or together with a solution of 1 µM ABA to the bottom of the plate. The suspension of the cells (25 µl/well) was added to the filter and the plate was incubated for 90 minutes at 37 °C. The migrated cells were transferred to a white 96-well plate and were counted with 60 µl of a solution containing 0.2 % (v/v) of Nonidet P40 and 1 µM Sitox green. After a 20-minute incubation at 37 °C, the fluorescence was measured with Fluostar OPTIMA fluorometer (excitation wavelength λ_{exc} = 485 nm and emission wavelength λₑₘᵢₛ = 520 nm) and the cell number was computed by using a standard cellular curve obtained from serial diltion cellular suspension of granulocytes. The result is expressed as the "chemotactic index" (CI), which corresponds to the ratio of the number of cells migrated towards the chemoattractant divided by the number of cells migrated towards the substrate). A chemotactic index higher than 1 is indicative of chemoattraction of cells. The test was carried out three times in sextuplicate.

As already described (Bruzzone, jbc 2007), ABA is capable of effectively promoting the migration of granulocytes, in fact the chemotactic index (CI) relatively to 1µM ABA is of 1,63.

The compound of example 1 was tested for inhibition of migration induced by 1 µM ABA at concentrations as shown in table 3 and, from the obtained result, it can be concluded that said compound is still effective at a concentration of 0.1 nM with an IC₅₀ of approximately 1 nM.

**TABLE 3. Inhibition of granulocyte migration towards ABA by compound of Example 1**

| | CI±SD | Inhibition % |
|---|---|---|
| Substrate | 1 | |
| ABA 1µM | 1.63±0.10 | |
| ABA 1 µM/Comp. from Ex. 1 5 µM | 0.78±0.03 | 100 |
| ABA 1 µM /Comp. from Ex. 1 100 nM | 1.12±0.04 | 80 |
| ABA 1 µM /Comp. from Ex. 1 10 nM | 1.26±0.07 | 57 |
| ABA 1 µM /Comp. from Ex. 1 1 nM | 1.32±0.05 | 47 |
| ABA 1 µM /Comp. from Ex. 1 0.1 nM | 1.37±0.05 | 38 |

### Example 6 - Production of reactive oxygen species wuith compound prepared according to example 1.

Human granulocitytes, after ON incubation in complete DMEM/F12, were resuspended at 1x10⁷/ml in HBSS and spiked with the fluorescent probe (10 µM), specific for ROS determination, 2',7'-dichlorodihydrofluorescein diacetate (H2DCFDA), for 30 min at 37 °C.

They were subsequently washed twice with HBSS and resuspended at 1x10⁶ cells/ml in HBSS and then were kept at room temperature for approximately 30 minutes.

The cells (100 µl) were added to a 96-well plate pre-loaded with ABA 1 µM, either alone or with increasing concentrations of the compound prepared according to example 1.

The increase in fluorescence was measured at 10-sec intervals for 10 minutes, with Fluostar OPTIMA fluorometer (λ_{exc}: 485; λₑₘᵢₛ: 520).

The experiment was carried out three times in triplicate.

Human granulocutes stimulated with 1 µM ABA become activated, and produce quite large amounts of reactive oxygen species (ROS) (Bruzzone, jbc 2007). The results were expressed as the average of the results obtained from three independent experiments, from which the background value, obtained from the control, had been subtracted.

**TABLE 4. Inhibition of ROS production, in human granulocytes stimulated with ABA, by compound prepared according to example 1.**

| | Increase in fluorescence (delta OD)±SD | Inhibition % |
|---|---|---|
| ABA 1 µM | 1238±8.7 | |
| Comp. of ex. 1 1 µM/ABA 1 µM | 46±0.2 | 96 |
| Comp. of ex. 1 1 nM/ABA 1 µM | 371±1.9 | 70 |
| Comp. of ex. 1 0.5 nM/ABA 1 µM | 680±13.6 | 45 |
| Comp. of ex. 1 0.01 nM/ABA 1µM | 1325±26.5 | 0 |

From the results reported in table 1, it can be concluded that the compound prepared according to example 1 is capable of inhibiting the production of ROS still at a concentration of 5 nM, with an IC₅₀ which can be easily deduced to be of approximately 0.5 nM.

### Example 7 - Inhibition of phagocytosis by compound prepared according to example 1.

Human granulocytes, after ON incubation in complete DMEM/F12, were resuspended at 1x10⁶ cells/100 µl in HBSS.

For the experiment, fluorescent polystyrene (carboxylate-modified polystyrene) beads were used, of 1 µm of diameter (Sigma-Aldrich) A volume of 100 µl of cellular suspension was subdivided into eppendorf, in which a 10 minutes long preincubation was carried out with 20 µM ABA, either alone, or together with increasing concentrations of compound prepared according to example 1.

After the end of the preincubation, the beads were added for 5, 10 and 30 minutes. For each experimental point, 2.5 x 10⁵ beads were used.

At the end of the incubations, the cells were collected by centrifugation (200xg for 10 seconds), were washed with 1 ml of HBSS at 0 °C and were centrifuged once again at 200xg for 10 seconds. The supernatant was discarded and the cells were resuspended in 350 µl of HBSS and added to 96-well plates of white colour (100 µl per well).

The increase in fluorescence was measured with Fluostar OPTIMA fluorometer (λ_{exc}: 485; λₑₘᵢₛ: 520).

The experiment was carried out three times in triplicate.

Human grnulocytes were preincubated for 10 minutes at 20 °C with 20 µM ABA, either alone or in the presence of compound prepared according to example 1, at various concentrations. In order to evaluate the phagocytic capability of granulocytes, fluorescent beads were added and, after 15 and 30 minutes, the fluorescence absorbed by the cells was determined as described in Materials and Methods The results were expressed as the average of the results obtained from three independent experiments, from which the background value, obtained from the control, had been subtracted (TABLE 5).

**TABLE 5. Inhibition of phagocitosis, in human granulocytes stimulated with ABA, by compound prepared according to example 1.**

| | Increase in fluorescence ±SD (@ 15 min) | Increase in fluorescence ±SD (@ 30 min) | Inhibition % (@ 15 min) | Inhibition % (@ 30 min) |
|---|---|---|---|---|
| ABA 20 µM | 909±47 | 2476±50 | | |
| ABA 20 µM / compound of example 1 | 17 | 0 | 98 | 100 |
| 1 µM | | | | |
| ABA 20 µM / compound of example 1 | 148±2 | 515±10 | 84 | 80 |
| 10 nM | | | | |
| ABA 20 µM / compound of example 1 | 210±2 | 1423±3 | 77 | 43 |
| 0.5 nM | | | | |

From the above data, it appears that the compound prepared according to example 1 has the capability of effectively inhibing phagocytosis induced by 20 µM ABA, also after a 30-minutes long incubation. From the obtained values, as resported in table 5, the IC₅₀ can be deduced, and results to be of approximately 0.5 nM.

### Example 8 - Inhibition of production of prostaglandins E2 (PGE2) in human granulocytes by compound prepared according to example 1

Granulocytes, in a number of 1x10⁷ per point in RPMI supplemented with 10 % of foetal calf serum (FCS), penicillin (50 units/ml) and streptomycin (50 µg/ml) (complete RPMI), were incubated for 6 h with 100 n ABA either alone or in the presence compound prepared according to example 1 at a concentration of 100 nM or of 1 µM. At the end of the incubation, the substrate was centrifuged at 17,000xg for 5 minutes and the concentration of PGE2 was quantitated by by using the kit: PGE2 monoclonal EIA kit (Cayman Chemical Co.), following the instructions suppled by the manufacturer.

The cells were lysed in 200 µl of deionized water in order to quantitate total proteins by the Bradford method (Bradford, M. M., 1976), after thre preliminary generation of a reference standard curve, obtained with different concentrations of bovine serum albumin. The spectrophotometric measurements were carried out at wavelength of 595 nm.

The experiment was carried out three times in triplicate TABLE 6).

**TABLE 6. Inhibition of PGE₂ production, in human granulocytes stimulated with ABA, by compound prepared according to example 1**

| | Relative amount of PGE₂ as referred to control ± SD | Inhibition % |
|---|---|---|
| Control | 1 | |
| ABA 100 nM | 1.6±0,37 | |
| ABA 100 nM + Comp. from Ex. 1 1 µM | 1.0±0,1 | 100 |
| ABA 100 nM + Comp. from Ex. 1 100 nM | 1.09±0,02 | 85 |

This test shows that compound prepared according to example 1 at a concentration of 1 µM is capable of inhibiting the ABA-induced production of PGE2 in granulocytes, with an inhibition rate of 100 %.

### Example 9 - Inhibition of production of prostaglandins E2 (PGE2) by compound prepared according to example 1

Monocytes, 25 x 10⁶/Petri in complete RPMI, were incubated for 6 h with 100 nM ABA either alone, or in the presence of compound prepared according to example 1, at a concentration of 1 µM. At the end of the incubation, the substrate was centrifuged at 17,000xg for 5 minutes and the concentration of PGE2 was quantitated by by using the kit: PGE2 monoclonal EIA kit (Cayman Chemical Co.), following the instructions suppled by the manufacturer.

The cells were lysed in 200 µl of deionized water in order to quantitate total proteins by the Bradford method (Bradford, M. M., 1976), after thre preliminary generation of a reference standard curve, obtained with different concentrations of bovine serum albumin. The spectrophotometric measurements were carried out at wavelength of 595 nm.

The experiment was carried out three times in sextuplicate.

The results were expressed as the average of the results from three independent experiments, as referred to control value. It was decided to test the compound prepared according to example 1 at the concentration of 1 µM only, in order to verify whether the inhibition rate of 100 %, as observed in the tests with human granulocytes, was still showed, even if in a different test, in a different cellular type as, in this case, human monocytes (TABLE 7).

**TABLE 7. Inhibition of PGE2 production, in human monocytes stimulated with ABA, by compound prepared according to example 1**

| | Relative amount of PGE₂ as referred to control ± SD | Inhibition % |
|---|---|---|
| Control | 1 | |
| ABA 100 nM | 2,2±0,2 | |
| ABA 100 nM + Comp. from Ex. 1 1 µM | 1,0±0,1 | 100 |

From this test it can be concluded that the compound prepared according to example 1 is capable of causing 100 % inhibition of ABA-induced production of PGE-2 in monocytes.

### Example 10 - Inhibition of MCP-1 (monocyte chemotactic protein) by compound prepared according to example 1.

Monocytes, 25 x 10⁶/Petri in complete RPMI, were incubated for 6 h with 100 nM ABA either alone, or in the presence of compound prepared according to example 1, at a concentration of 1 µM. At the end of the incubation, the substrate was centrifuged at 17,000xg for 5 minutes and the release of MCP-1 (monocyte chemotactic protein-1) was quantitated by means of kit MC=-1 Elisa kit (GE Healthcare), in accordance with the instructions supplied by the manufacturer.

The cells were lysed in 200 µl of deionized water in order to quantitate total proteins by the Bradford method, after thre preliminary generation of a reference standard curve, obtained with different concentrations of bovine serum albumin. The spectrophotometric measurements were carried out at wavelength of 595 nm.

The experiment was carried out three times in sextuplicate.

The results were expressed as the average of the results from three independent experiments, as referred to control value. It was decided to test the compound prepared according to example 1 at the concentration of 1 µM only, in order to verify whether the inhibition rate of 100 %, as observed in the tests with human granulocytes, was still showed, even if in a different test, in a different cellular type as, in this case, human monocytes.

**TABLE 8. Inhibition of MCP-1 production in human monocytes by compound prepared according to example 1**

| | MCP-1 relatively to control ± SD | Inhibition % |
|---|---|---|
| Control | 1 | |
| ABA 100 nM | 2±0.2 | |
| ABA 100 nM + Comp. from Ex. 1 1 µM | 0.8±0.2 | 100 |

From this test it can be concluded that the compound prepared according to example 1 is capable of causing 100 % inhibition of ABA-induced production of MCP-1 in monocytes.

### Example 11 - Test of stability of compound prepared according to example 1.

Compound prepared according to example 1 was tested for stability at 10 µM concentration in deionized water, phosphate buffer (HBSS with calcium and magnesium) and human plasma for 0 and after 24 hrs at 37 °C, by carrying out the extraction as described in Materials and Methods, and subsequent HPLC-MS analysis for the analyte of interest.

Additionally, stability tests were carried out in water, in HBSS and in HBSS in the presence of human granulocytes at time 0, and after 1 and 2 hrs at 37 °C, and carrying out HPLC-MSS analysis without extracting the analyte of interest.

For stability tests, to 2 ml of deionized water, HBSS or human plasma, the compound of example 1 (10 µM) and 5,000 cpm of [³H]ABA were added, for the purpose of evaluating the recovery after the extraction procedure. The so prepared samples were exctracted at time 0 and after 24 hrs.

For the extraction, 8 ml was added of distilled methanol (methanol/water : 80/20 vol/vol) and the mixture was kept at -20 °C overnight.

The following day the samples were centrifuged at 800xg for 10 minutes and the supernatant was concentrated to dryness by Rotavapor. The reconstituted aqueous solution was acidified at pH 2.6 with 10 % trifluoroacetric acid (TFA) and was extracted three times with diethyl ether (diethyl ether/water: 1:1 vol/vol).

The ethereal phase was finally removed by Rotavapor and the extraxt was resuspended in 1 ml of HBSS; 8 µl of such solution was analysed by HPLC-MS, and 100 µl was used for radioactivity counting by a Packard β-counter in order to compute extraction recovery.

In order to test the compound prepared according to example 1 for stability in HBSS in the presence, or absence, of granulocytes, 1 x 10⁸ cells were used in 2 ml of HBSS (with calcium and magnesium added) containing 10 µM of compound prepared according to example 1 at 37°C. Samples of 500 µl each were drawn at time 0, and after 1 and 2 hrs and the cells were separated by centrifugation at 200xg for 5 minutes; the supernatant was then recovered and directly analysed in HPLC-MS.

The HPLC-MS system used consists of an Agilent 1100 capillary chromatograph equipped with a diode array detector (DAD) and coupled with an Agilent 1100 series LC/MSD Trap mass spectometer with orthogonal electrospray source and a ionic-trap analyser.

The detection wavelength was 254 nm and the MS spectrum was recorded in negative ions mode in an m/z range of 50-300.

The amount of compound of example 1 in the extracts and supernatants was computed as the area of the peak under the extracted ion current m/z 279.3, normalized for the ionization efficiency of internal standard (hexadeuterated ABA) used for HPLC-MS analysis. Furthermore, the concentration was computed using the recovery rates obtained from the evaluation of counts per minute (cpm) of extracted [³H]ABA.

The data was obtained of stability of compound prepared according to example 1 after extraction from H₂O, HBSS and plasma at time 0 and after 24 hrs, and of compound prepared according to example 1 (without extraction), H₂O, HBSS and on granulocytes at time 0, then after 1 hr and 2 hrs.

As it can be observed from table 9, from the comparison of the sample extracted at 0 and after 24 hrs, the compound prepared according to example 1 appears to undergo a degradation of 14 % in water, of 3 % in phosphate buffer and of 7 % in plasma. Therefore, it can be concluded that the compound from example 1 is stable in the tested environments. Furthermore, from table 9 it can be observed that the extraction procedure yields 100 % and 76 % in water and respectively phosphate buffer, and 56 % in plasma. In table 10 the stability is shown of compound prepared according to examples 1 incubated in phosphate buffer with human granulocytes, and from the comparison of non-extracted samples, a degradation rate of compound from example 1 of about 10 %/ hour can be inferred.

**Table 9: Stability of compound of example 1 after extraction from H₂O, HBSS and PLASMA at time 0 and after 24 hrs**

| Media | 0 hr | 24 hrs |
|---|---|---|
| H₂O | 100% | 86% |
| HBSS | 76% | 73% |
| Plasma | 56% | 49% |

**Table 10: Stability of compound of example 1 (without extraction) in H₂O, HBSS and PLASMA at time 0, and after hr and 24 hrs**

| | 0 hr | 1 h | 2 hrs |
|---|---|---|---|
| H₂O | 100% | 100% | 98% |
| HBSS | 100% | 100% | 89% |
| Granulocytes | 94% | 82% | 73% |

### Example 12 - Test of toxicity of compound prepared according to example 1.

The possible cytotoxicity of compound from example 1 was evaluated on:
1) A cellular line of human astrocytoma (1321N1) cultured in Dulbecco's modified Eagle's medium (DMEM) supplemented with 10 % of foetal calf serum (FCS), penicillin (50 units/ml) and streptomycin (50 µg/ml) and kept under humidified atmosphere at 37 °C with 5% di CO₂.

The cells (5x10³ cells/well) were treated in quadruplicate in 200 µl with different concentrations of compound prepared according to example 1 (1 µM; 100 nM; 10 nM; 1 nM) for 24 hrs at 37 °C.

Human granulocytes separated from healthy donors cultured in Dulbecco's modified Eagle's medium: F12 (Dmem/F12) supplemented with 5 % of foetal calf serum (FCS) and kept under a humidified atmosphere at 37°C with CO₂ 5 %.

The cells (20 x 10⁴ cells/well) were treated in quadruplicate in 200 µl with different concentrations of compound prepared according to example 1 (1 µM; 100 nM; 10 nM; 1 nM) for 24 hrs at 37 °C.
3) A cellular line of murine monocytic leukemia (Raw 264.7) cultured in Dulbecco's modified Eagle's medium (DMEM) supplemented with 10 % of foetal calf serum (FCS) and kept under humidified air at 37 °C with 5% di CO₂.

The cells (15 x 10⁴ cells/well) were treated in quadruplicate in 200 µl with different concentrations of compound prepared according to example 1 (1 µM; 100 nM; 10 nM; 1 nM) for 24 hrs at 37 °C.

At the end of the 24 hrs-time, 40 µl of CellTiter 96® AQueous One Solution Cell Proliferation Assay (Promega) were added to the plates, and the cells were incubated for 90 minutes at 37 °C in the dark.

The cell number was computed by using a standard cell curve, obtained from serial dilution of the various cell suspensions.

The absoption was read at 490 nm with a 96 well-plate reader (BIORAD model 40).

The toxicity rate was calculated relatively to untreated control by subtracting the reading obtained from substrate alone per each tested concentration of compound prepared according to example 1.

As it can be observed from table 11, the compound prepared according to example 1 does not result to be toxic at any tested concentration, nor does it seem to reduce the proliferative capability of the tested cells.

**TABLE 11. Cytotoxicity of compound prepared according to example 1 in different cell lines**

| 24 hrs | 1321 N1 (human astrocytoma) | | Human granulocytes | | Raw 264.7 (murine monocitic leukemia) | |
|---|---|---|---|---|---|---|
| | vitality % | number of cells | vitality % | number of cells | vitality % | number of cells |
| Control | 100 | 18348 | 100 | 188221 | 100 | 305541 |
| Compound of Ex. 1, 1 µM | 106 | 19446 | 83 | 132555 | 90 | 274987 |
| Compound of Ex. 1, 100 µM | 120 | 22252 | 96 | 175388 | 95 | 290264 |
| Compound of Ex. 1, 10 nM | 102 | 18785 | 100 | 191138 | 100 | 30555 |
| Compound of Ex. 1, 1 nM | 89 | 16199 | 89 | 151138 | 100 | 306321 |

### Example 13 - Effect on insulin release by INS-1 cells of compound prepared according to example 1 and of ABA

Insulinoma cells of INS-1 rat were transfected with LANCL-2 silencing RNAs LANCL-2 (siRNAL2) or with nonsense RNA (scr) and were incubated in 5 mM glucose (LG) oppure 25 mM glucose (HG), in the absence or in the presence of 100 nM ABA or of compound according to example 1 (100 nM). Insulin release is expressed as the secretion index (SI) relative to insulin release in LG. The results are given in table 12. As regards the experimental procedure, with the exception of use of compound according to example 1, reference can be made to the following paper, in which the properties which are the subject-matter of the present example were investigated for only ABA. Sturla L, Fresia C, Guida L, Grozio A, Vigliarolo T, Mannino E, Millo E, Bagnasco L, Bruzzone S, De Flora A, Zocchi E. Binding of abscisic acid to human LANCL2. Biochem Biophys Res Commun. 2011 Nov 18;415(2):390-5.

**TABLE 12. Effect of ABA and of compound from example 1 on insulin release (SI) by INS-2 cells at 30 minutes.**

| | SCR | LANCL2 siRNA |
|---|---|---|
| LG | 1 | 1 |
| HG | 4 | 1.9 |
| LG + ABA 100 nM | 1.7 | 1 |
| HG + ABA 100 nM | 6.4 | 1.7 |
| LG + compound from Ex. 1 100 nM | 2.5 | 2 |
| HG + compound from Ex 1 100 nM | 6 | 3 |
| LG+ABA 100 nM + compound from Ex 1 100 nM | 8.5 | 5 |
| HG+ABA 100 nM + compound from Ex 1 100 nM | 12 | 8 |

### Example 14 - Effect on insulin release by INS-1 cells of compound prepared according to example 1 and of ABA

Human pancreatic beta cells were incubated in 5 nM glucose (LG), or 25 mM glucose (HG), in the absence or in the presence of 100 nM ABA or of compound according to example 1 (100 mM). Insulin release is expressed as the secretion index (SI) relative to insulin release in LG.. The results are given in table 13.

As in the preceding example, for the experimental procedere, reference may be made to the there cited paper, except for the specific use of compound according to example 1.

**TABLE 13. Effect of ABA and of compound prepared according to example 1 on insulin release (SI) by human pacreatic beta cells at 30 minutes and 120 minutes.**

| | SI |
|---|---|
| LG | 1 |
| HG at 30 minutes | 7,2 |
| LG + (ABA 100 nM) at 30 minutes | 0,91 |
| LG + compound from Ex. 1 100 nM at 30 minutes | 1,8 |
| LG + ABA (100 nM) + compound from Ex 1 (100 nM) | 0,72 |
| HG at 120 minutes | 1,1 |
| LG + ABA 100 nM at 120 minutes | 1,8 |
| LG + compound from Ex 1 100 nM at 120 minutes | 2,2 |
| HG + ABA (100 nM) + compound from Ex 1 (100 nM) | 3,8 |

## Claims

1. Compound of formula (I) 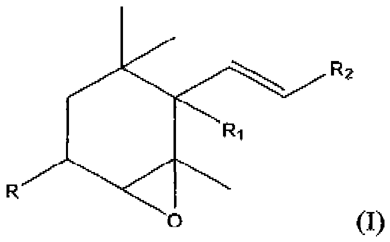 wherein R is selected from hydrogen e =O groups, R1 is selected from -H e -OH, R2 is selected from groups having one of following structural formulae (II), (III): 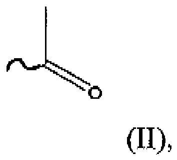 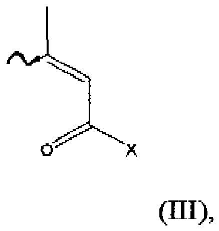 wherein X is selected from -OH, alkylamino-, arylamino-, alkylarylamino- or arylalkylamino-, alkoxy-, aryloxy-, alkylaryloxy- or arylalkoxy- groups, with said groups having from one to eight carbon atoms, with the alkyl chains of such groups being optionally branched, containing unsaturations or forming rings, salts of said compound or pharmaceutically acceptable hydrates or solvates thereof, for use as a drug.

2. Compound according to claim 1, wherein X shows keto, carboxy, ether and/or amino functionalities, or pharmaceutically acceptable salts, hydrates or solvates thereof, for use as a drug.

3. Compound according to any of preceding claims, or pharmaceutically acceptable salts, hydrates or solvates thereof for use as an antiinflammatory drug, in particular for treatment of atherosclerosis, metabolic syndrome, cronic inflammatory pathologies.

4. Compound according to claim 1 or 2, or pharmaceutically acceptable salts, hydrates or solvates thereof, for use as a drug for treatment of type 2 diabetes.

5. Compound according to any of preceding claims, or pharmaceutically acceptable salts, hydrates or solvates thereof, for use as a drug for human or veterinary use.

6. Compound according to any preceding claim, said compound being the compound of formula (I), wherein R is hydrogen, R1 is hydrogen, R2 corresponds to formula (II), or pharmaceutically acceptable salts, hydrates or solvates thereof, for use as a drug.

7. Compound according to any of claims from 1 to 4, said compound being 2'α,3'α-dihydro-2'α,3'α-epoxyabscisic acid or pharmaceutically acceptable salts, hydrates or solvates thereof, for use as a drug.

8. Compound according to any of claims from 1 to 4 or pharmaceutically acceptable salts, hydrates or solvates thereof, wherein R is =O, R1 is -OH, R2 corresponds to formula (III), with X being selected from -OCH₃, -NHCH₃, -N(CH₂CH₃)₂, or any of the following structural formulae (IV), (V), (VI), (VII): 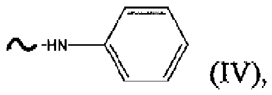 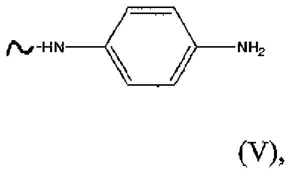 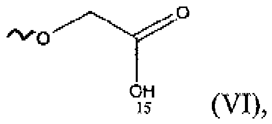 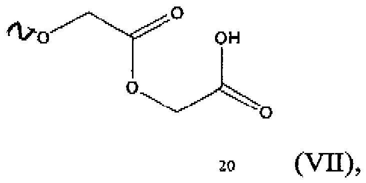 for use as a drug.

9. Compound according to any of preceding claims, wherein the epoxy functionality is inserted in *trans* configuration, or pharmaceutically acceptable salts, hydrates or solvates thereof, for use as a drug.

10. Compound according to any of preceding claims as a mixture of different stereosisomers, or pharmaceutically acceptable salts, hydrates or solvates thereof, for use as a drug.

11. Compound according to any of preceding claims, or pharmaceutically acceptable salts, hydrates or solvates thereof, wherein, relatively to the C-C double bond contained in formula (I), the substituents are arranged in E configuration and, relatively to the double C-C bond contained in formula (III), if present, the substituents are arranged in Z configuration for use as a drug.

12. Pharmaceutical composition comprising a compound according to any of preceding claims, or pharmaceutically acceptable salts, hydrates or solvates thereof, and one or more pharmaceutically acceptable excipients or solvents, for use as a drug.

13. Pharmaceutical composition comprising a compound according to claim 4, or pharmaceutically acceptable salts, hydrates or solvates thereof. and one or more pharmaceutically acceptable excipients or solvents and abscisic acid, for use as a drug.

## Patentansprüche

1. Verbindung der Formel (I) 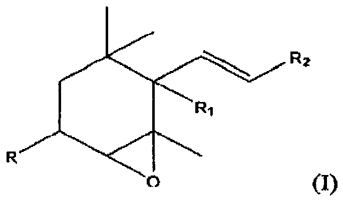 wobei R aus den Wasserstoff- und =0-Gruppen ausgewählt ist, R1 aus den -H- und -OH-Gruppen, R2 aus den Gruppen mit einer der folgenden Strukturformeln (II), (III) ausgewählt ist: 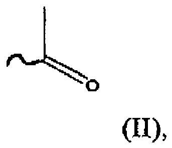 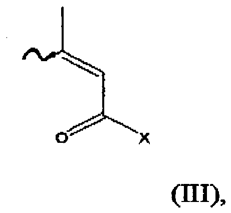 wobei X aus den -OH-, Alkylamin-, Arylamin-, Alkylarylamin- oder Arylakylamin-, Alkoxy-, Aryloxy-, Alkylaryloxy- oder Arylalkoxy-Gruppen ausgewählt ist, wobei die genannten Gruppen ein bis acht Kohlenstoffatome besitzen, wobei gegebenenfalls die Alkylketten der genannten Gruppen verzweigt sind, Ungesättigtheiten enthalten oder Ringstrukturen bilden, Salzen der genannten Verbindung oder davon pharmazeutisch geeigneten Hydraten oder Solvaten zur Verwendung als Arzneimittel.

2. Verbindung nach Anspruch 1, worin X Keto-, Carboxy-, Äther- und/oder Aminfunktionen aufweist oder davon pharmazeutisch geeignete Salze, Hydrate oder Solvate zur Verwendung als Arzneimittel.

3. Verbindung nach einem der vorangegangenen Ansprüche oder davon pharmazeutisch geeignete Salze, Hydrate oder Solvate zur Verwendung als entzündungshemmendes Arzneimittel, insbesondere zur Behandlung von Arteriosklerose, metabolischem Syndrom, chronischen Entzündungskrankheiten.

4. Verbindung nach Anspruch 1 oder 2 oder davon pharmazeutisch geeignete Salze, Hydrate oder Solvate zur Verwendung als Arzneimittel zur Behandlung der Typ-2-Diabetes.

5. Verbindung nach einem der vorangegangenen Ansprüche oder davon pharmazeutisch geeignete Salze, Hydrate oder Solvate zur Verwendung als Arzneimittel in der menschlichen und Tiermedizin.

6. Verbindung nach einem der vorangegangenen Ansprüche, wobei die genannte Verbindung die Verbindung der Formel (I) ist, wobei R Sauerstoff ist, R1 Sauerstoff ist, R2 der Formel (II) entspricht oder davon pharmazeutisch geeignete Salze, Hydrate oder Solvate zur Verwendung als Arzneimittel.

7. Verbindung nach einem der Ansprüche von 1 bis 4, wobei die Verbindung eine 2'α,3'α-dihydro-2'α,3'α-Epoxy-Abszisinsäure ist oder davon pharmazeutisch geeignete Salze, Hydrate oder Solvate zur Verwendung als Arzneimittel.

8. Verbindung nach einem der Ansprüche von 1 bis 4 oder davon pharmazeutisch geeignete Salze, Hydrate oder Solvate, wobei R =O ist, R1 -OH ist, R2 der Formel (III) entspricht, mit X ausgewählt aus -OCH₃, -NHCH₃, -N(CH₂CH₃)₂ oder eine der folgenden Strukturformeln (IV), (V), (VI), (VII): 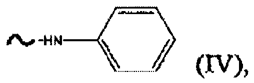 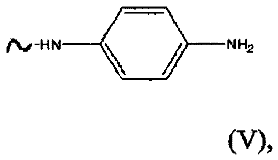 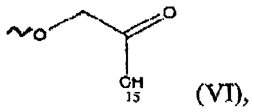 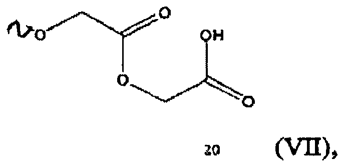 zur Verwendung als Arzneimittel.

9. Verbindung nach einem der vorangegangenen Ansprüche, wobei die Epoxyfunktion in eine *trans-*Konfiguration eingefügt ist oder davon pharmazeutisch geeignete Salze, Hydrate oder Solvate zur Verwendung als Arzneimittel.

10. Verbindung nach einem der vorangegangenen Ansprüche als eine Mischung verschiedener Stereoisomere oder davon pharmazeutisch geeignete Salze, Hydrate oder Solvate zur Verwendung als Arzneimittel

11. Verbindung nach einem der vorangegangenen Ansprüche oder davon pharmazeutisch geeignete Salze, Hydrate oder Solvate, wobei bezüglich der in der Formel (I) enthaltenen C-C-Doppelbindung die Substituenten in E-Konfiguration angeordnet sind und, bezüglich der in der Formel (III) enthaltenen C-C-Doppelbindung, falls vorhanden, die Substituenten in Z-Konfiguration angeordnet sind, zur Verwendung als Arzneimittel.

12. Pharmazeutische Zusammensetzung umfassend eine Verbindung nach einem der vorangegangenen Ansprüche oder davon pharmazeutisch geeignete Salze, Hydrate oder Solvate, und einen oder mehrere pharmazeutisch geeignete Arzneistoffsträger oder Lösungsmittel, zur Verwendung als Arzneimittel.

13. Pharmazeutische Zusammensetzung umfassend eine Verbindung nach Anspruch 4 oder davon pharmazeutisch geeignete Salze, Hydrate oder Solvate, und einen oder mehrere pharmazeutisch geeignete Arzneistoffsträger oder Lösungsmittel und Abszisinsäure, zur Verwendung als Arzneimittel.

## Revendications

1. Composé de formule (I) 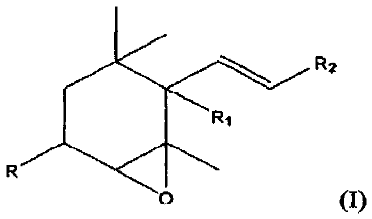 où R est choisi parmi des groupes hydrogène et =O, R₁ est choisi entre -H et -OH, R₂ est choisi parmi des groupes ayant une des formules structurales (II), (III) suivantes: 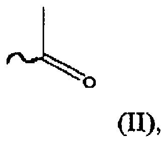 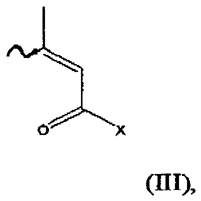 où X est choisi parmi -OH, des groupes alkylamino, arylamino, alkylarylamino ou arylalkylamino, alcoxy, aryloxy, alkylaryloxy ou arylalcoxy, lesdits groupes ayant un à huit atomes de carbone, où les chaînes alkylées de tels groupes sont facultativement ramifiées, contiennent des unsaturations ou forment de structures cycliques, sels du composé nommé précédemment, ou ses hydrates ou solvates pharmaceutiquement acceptables, pour son utilisation comme médicament.

2. Composé selon la revendication 1, où X comporte des fonctions céto, carboxy, éther, amine, ou ses sels, hydrates ou solvates pharmaceutiquement acceptables, pour son utilisation comme médicament.

3. Composé selon l'une quelconque des revendications précedentes, ou ses sels, hydrates ou solvates pharmaceutiquement acceptables pour son utilisation comme un médicament anti-inflammatoire, en particulier pour le traitement de artériosclérose, syndrome métabolique, pathologies inflammatoires chroniques.

4. Composé selon les revendications 1 ou 2, ou ses sels, hydrates ou solvates pharmaceutiquement acceptables pour son utilisation comme un médicament anti-inflammatoire, en particulier pour le traitement de diabète de type II.

5. Composé selon l'une quelconque des revendications précedentes, ou ses sels, hydrates ou solvates pharmaceutiquement acceptables pour son utilisation comme médicament pour l'homme ou vétérinaire.

6. Composé selon l'une quelconque des revendications précedentes, ledit composé étant le composé de formule (I), où R est hydrogène, R₁ est hydrogène, R₂ correspond à la formule (II), ou ses sels, hydrates ou solvates pharmaceutiquement acceptables, pour son utilisation comme médicament.

7. Composé selon l'une quelconque des revendications 1 à 4, ledit composé étant acide 2'α,3'α-dihydro-2'α,3'α-époxyabscissique ou ses sels, hydrates ou solvates pharmaceutiquement acceptables, pour son utilisation comme médicament.

8. Composé selon l'une quelconque des revendications 1 à 4, ou ses sels, hydrates ou solvates pharmaceutiquement acceptables, où R est =OH, R₁ est -OH, R₂ correspond à la formule (III), où X est choisi parmi -OCH₃, -NHCH₃, -N(CH₂CH₃)₂, ou l'une quelconque des formules structurales (IV), (V), (VI), (VII): 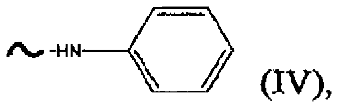 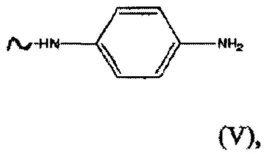 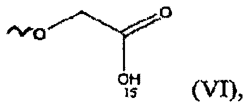 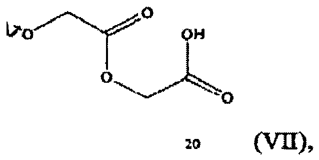 pour son utilisation comme médicament.

9. Composé selon l'une quelconque des revendications précedentes, où la fonction époxy est introduite en configuration trans, ou ses sels, hydrates ou solvates pharmaceutiquement acceptables, pour son utilisation comme médicament.

10. Composé selon l'une quelconque des revendications précedentes, comme un mélange de stéréisomères différents, ou ses sels, hydrates ou solvates pharmaceutiquement acceptables, pour son utilisation comme médicament.

11. Composé selon l'une quelconque des revendications précedentes, ou ses sels, hydrates ou solvates pharmaceutiquement acceptables, où, pour ce qui concerne la double liaison C-C contenue dans la formule (I), les substituants sont arrangés en configuration E, tandis que, pour ce qui concerne la double liaison C-C contenue dans la formule (III), si présente, les substituants sont arrangés en configurazion Z, pour son utilisation comme médicament.

12. Composition pharmaceutique comprenant un composé selon l'une quelconque des revendications précedentes, ou ses sels, hydrates ou solvates pharmaceutiquement acceptables, ainsi qu'un ou plusiers excipients ou solvants pharmaceutiquement acceptables, pour son utilisation comme médicament.

13. Composition pharmaceutique comprenant un composé selon la revendication 4, ou ses sels, hydrates ou solvates pharmaceutiquement acceptables, ainsi qu'un ou plusiers excipients ou solvants pharmaceutiquement acceptables et de l'acide abscissique, pour son utilisation comme médicament.
